# EUROPEAN PATENT APPLICATION

(11) **EP 4 835 097 A1**
(43) Date of publication of application: **04.11.2026**
(21) Application number: 24913104.6
(22) Date of filing: 27.12.2024
(51) Int. Cl.: C12Q 1/686, C12N 1/15, C12N 1/19, C12N 1/21, C12N 5/10, C12N 15/10, C12N 15/12, C12N 15/54, C12N 15/63, C12Q 1/6883, G01N 33/53

(54) **METHOD FOR PRODUCING GENOMIC DNA AS REFERENCE SUBSTANCE FOR CALIBRATION USED IN ANALYSIS OF MUTATION, AND METHOD FOR ANALYZING GENOMIC DNA, CELL, AND TARGET SEQUENCE MUTATION**

(30) Priority: 28.12.2023 JP 2023223355
(71) Applicant: Denka Company Limited, Tokyo 103-8338 (JP)
(72) Inventor: KUBO, Hiroshi, Tokyo 103-8338 (JP)
(74) Representative: Müller-Boré & Partner Patentanwälte PartG mbB
(86) International application number: PCT/JP2024/046387
(87) International publication number: WO 2025/143218

(57) **Abstract**

A method of producing genomic DNA as a calibration reference material to be used in analyzing a mutation in a target sequence, including a step of introducing a mutated target sequence and an internal standard sequence into genomic DNA.

## Description

### Technical Field

The present invention broadly relates to a method of producing genomic DNA as a calibration reference material to be used in mutation analysis, genomic DNA, a cell, and a method of analyzing a mutation in a target sequence.

### Background Art

A chemotherapeutic agent that targets an abnormal enzyme having an amino acid mutation is referred to as a molecularly targeted drug. In order to administer a molecularly targeted drug, it is necessary to first examine, using a diagnostic agent, the presence of the amino acid mutation, and such a diagnostic agent is referred to as a companion diagnostic agent. Among companion diagnostic agents, a diagnostic agent that examines an amino acid mutation indirectly by detecting a gene mutation is referred to as a gene mutation diagnostic agent.

The sensitivity of the gene mutation diagnostic agent, particularly, for example, the detection sensitivity of a mutant gene, is defined by a variant allele frequency (VAF), which is an index of the frequency of the mutant gene, and the detection sensitivity is evaluated using a calibration reference material in which the VAF is defined. For diagnosis standardization and quality control, it is necessary to use a common standard such as a calibration reference material, and plasmids, yeast, genomic DNA and the like are known as such materials (Non-Patent Literature 1 and 2).

As a method of determining the VAF of a calibration reference material, for example, a method using the copy number of a mutant gene in the calibration reference material measured by a droplet digital PCR (ddPCR) is known. When the copy number of the mutant gene is used, a validated droplet digital PCR reagent is required for each mutant gene. However, when a validated droplet digital PCR reagent for a target mutant gene is not commercially available, it is necessary for a user to directly design and validate probes for use in droplet digital PCR. This requires more effort as the number of types of target mutant genes increases.

### Citation List

### Non-Patent Literature

Non-Patent Literature 1: H White et al. A certified plasmid reference material for the standardization of BCR-ABL1 mRNA quantification by real-time quantitative PCR. Leukemia. 2015, 29, 369-376.
Non-Patent Literature 2: Xin He et al. Development of a new genetic reference material system based on Saccharomyces cerevisiae cells. Molecular Therapy: Methods & Clinical Development. 2021, 20, 473-482.

### Summary of Invention

### Technical Problem

In order to determine the VAF, it is necessary to accurately measure the copy number of the mutant gene, and among validated methods, ddPCR is the most reliable method in the current technology.

An object achieved by the present invention is to provide a novel method of producing a calibration reference material by which a VAF can be accurately calculated using ddPCR regardless of the copy number of a mutant gene. Another object achieved by the present invention is to provide a novel method of producing a calibration reference material by which the VAF can be easily calculated for a plurality of calibration reference materials having various mutant genes without preparing a validated droplet digital PCR reagent for each mutant gene.

### Solution to Problem

The inventors conducted extensive studies regarding a step of introducing a mutant gene into genomic DNA in the method of producing genomic DNA as a calibration reference material, and as a result, found a novel method of producing genomic DNA as a calibration reference material by which the VAF can be calculated without using the copy number of the mutant gene.

Specifically, this application includes the following inventions.
[1] A method of producing genomic DNA as a calibration reference material to be used in analyzing a mutation in a target sequence, including
   a step of introducing a mutated target sequence and an internal standard sequence into genomic DNA.
[2] The method according to [1], wherein the introduced target sequence and internal standard sequence are derived from a recombinant vector.
[3] The method according to [2], wherein the recombinant vector is a transposon vector.
[4] The method according to [2] or [3], wherein the recombinant vector is produced by a method including
   1) a step of cleaving a recombinant vector with a first restriction enzyme, wherein the recombinant vector has a nucleic acid sequence in which a first restriction enzyme recognition sequence, a second restriction enzyme recognition sequence, and a first restriction enzyme recognition sequence are arranged in that order,
      the two first restriction enzyme recognition sequences are arranged such that cleavage sites for two first restriction enzymes are located on the sides opposite to the second restriction enzyme recognition sequence, and
      the first restriction enzyme recognition sequence is a Type IIS restriction enzyme recognition sequence, and the second restriction enzyme recognition sequence is a restriction enzyme recognition sequence different from the first restriction enzyme recognition sequence; and
   2) a step of ligating a double-stranded nucleic acid containing a region composed of a polymorphic nucleotide in a target sequence and its 5'-side and 3'-side flanking sequences together with their complementary sequences.
[5] The method according to any one of [1] to [4], wherein the introduced sequence further contains a selection marker.
[6] The method according to any one of [1] to [5], wherein the mutation is a polymorphism.
[7] The method according to any one of [1] to [6], wherein the analysis is a companion diagnostic.
[8] The method according to any one of [1] to [7], wherein the calibration reference material is used to validate a companion diagnostic agent.
[9] Genomic DNA produced by the method according to any one of [1] to [8].
[10] A cell containing one or more copies of genomic DNA into which a mutated target sequence and an internal standard sequence have been introduced.
[11] The cell according to [10], wherein the chromosome number is diploid.
[12] A method of analyzing a mutation in a target sequence, including:
   1) a step of subjecting genomic DNA into which a mutated target sequence and an internal standard sequence have been introduced to an amplification reaction; and
   2) a step of calculating the copy numbers of the introduced internal standard sequence and a different internal standard sequence inherent to the genomic DNA.
[13] The method according to [12], further including a step of calculating a variant allele frequency (VAF) based on the copy number.
[14] The method according to [13],
   wherein the VAF is a percentage value calculated from the following formula: (copy number of introduced internal standard sequence-copy number of different internal standard sequence inherent to genomic DNA)/copy number of introduced internal standard sequence.
[15] The method according to any one of [12] to [14], which is used to evaluate the sensitivity of a reagent for detecting a mutation.
[16] The method according to [15], wherein the evaluation of the sensitivity is used as an index for validating the reagent for detecting the mutation.
[17] The method according to any one of [12] to [16], wherein the amplification reaction is performed by a PCR.
[18] The method according to [17], wherein the PCR is a droplet digital PCR.

### Advantageous Effect of Invention

According to the present invention, it is possible to produce genomic DNA as a calibration reference material, which enables a VAF to be calculated by using the copy number of an internal standard gene derived from genomic DNA without using the copy number of a mutant gene.

### Brief Description of Drawings

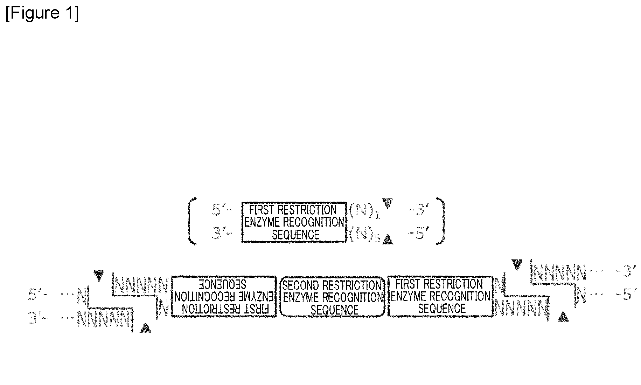

[Figure 1] Figure 1 is a schematic diagram showing the arrangement of a first restriction enzyme recognition sequence and a second restriction enzyme recognition sequence in a recombinant vector when the first restriction enzyme recognition sequence is cleaved at sites 1 to 5 nucleotides apart from the recognition sequence.

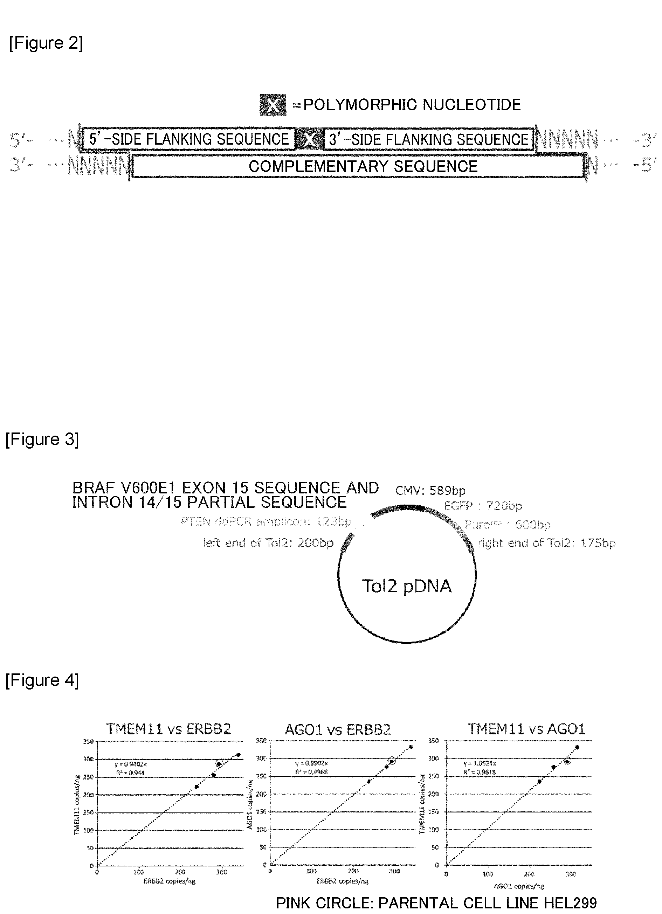

[Figure 2] Figure 2 is a schematic diagram showing a case in which a double-stranded nucleic acid is inserted and ligated after the treatment in the first restriction enzyme recognition sequence where the sites 1 to 5 nucleotides apart from the recognition sequence are cleaved.
[Figure 3] Figure 3 shows a structure diagram of ToI2 pDNA (SEQ ID NO: 1).
[Figure 4] Figure 4 shows the results of analyzing the copy numbers of the AGO1 gene, the ERBB2 gene, and the TMEM11 gene in HEL299 cells without gene introduction and HEL299 cells into which genes were introduced and stably incorporated in order to confirm the arbitrariness and robustness of a second internal standard gene.

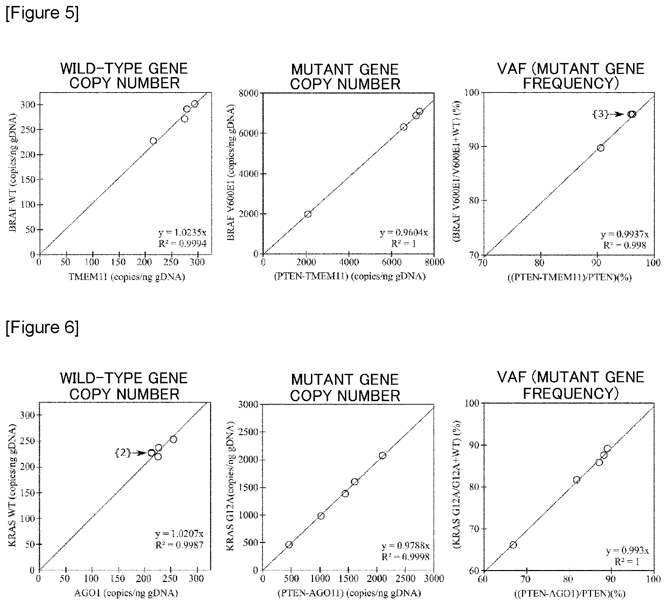

[Figure 5] The left panel of Figure 5 shows the relationship between the copy number of the BRAF WT gene and the copy number of the TMEM11 gene. The center panel of Figure 5 shows the relationship between the copy number of the BRAF V600E1 gene, and the value obtained by subtracting the copy number of the TMEM11 gene from the copy number of the PTEN gene. The right panel of Figure 5 shows the relationship between the VAF calculated from the copy number of the BRAF WT gene and the copy number of the BRAF V600E1 gene, and the VAF calculated from the copy number of the PTEN gene and the copy number of the TMEM11 gene.
[Figure 6] The left panel of Figure 6 shows the relationship between the copy number of the KRAS WT gene and the copy number of the AGO1 gene. The center panel of Figure 6 shows the relationship between the copy number of the KRAS G12A gene and the value obtained by subtracting the copy number of the AGO1 gene from the copy number of the PTEN gene. The right panel of Figure 6 shows the relationship between the VAF calculated from the copy number of the KRAS WT gene and the copy number of the KRAS G12A gene, and the VAF calculated from the copy number of the PTEN gene and the copy number of the AGO1 gene.

### Description of Embodiments

Hereinafter, an embodiment (hereinafter referred to as "the present embodiment") of the present invention will be described, but the scope of the present invention is not limited to the interpretation of the following embodiment.

### (Method of producing genomic DNA as calibration reference material to be used in analyzing mutation in target sequence)

A first embodiment provides a method of producing genomic DNA as a calibration reference material to be used in analyzing a mutation in a target sequence, including a step of introducing a mutated target sequence and an internal standard sequence into genomic DNA.

As used herein, the term "mutation" refers to one or more nucleotides that have undergone substitution, deletion or insertion of a nucleotide(s), or a state in which nucleotide substitution or the like has occurred. "Mutation" can be used interchangeably with "polymorphism." The positions of nucleotides that have undergone substitution, deletion or insertion of a nucleotide(s), the number of nucleotides to be substituted or deleted, and the number of nucleotides to be inserted can be appropriately set according to the purpose. The types of mutations are not particularly limited, and examples thereof include missense mutations, nonsense mutations, frameshift mutations, and silent mutations. In the present embodiment, the mutation may be a polymorphism.

As used herein, the term "calibration reference material" refers to a material that serves as a reference for evaluating an analysis method or a diagnostic agent or calibrating a device. The calibration reference material may also be referred to as a calibration standard material. In addition, as used herein, "genomic DNA as a calibration reference material used in analysis of mutations in a target sequence" broadly refers to genomic DNA having a mutant gene.

As used herein, the term "genomic DNA" refers to DNA that constitutes the entire set of genetic information (genome) of cells.

As used herein, the term "internal standard sequence" refers to any gene sequence derived from genomic DNA into which a mutated target sequence and an internal standard sequence are introduced, which is different from the target sequence, but is preferably an internal standard sequence for which a validated droplet digital PCR reagent is commercially available. In addition, a gene sequence in a region near a centromere, where transposon transition or viral gene insertion is unlikely to occur, is preferable. The target sequence and the internal standard sequence may be sequences derived from different gene loci or may be sequences derived from different chromosomes. There is no need to select, as the internal standard sequence, a sequence of a housekeeping gene or the like, which is constitutively expressed in cells and has small expression variation.

Examples of internal standard sequences include the PTEN gene, the AGO1 gene, the TMEM11 gene, and the ERBB2 gene.

As used herein, the term "droplet digital PCR" refers to a digital PCR technique that measures an absolute amount of a target sequence by counting nucleic acid molecules encapsulated in volumetrically homogenized water-in-oil emulsion droplets. Unlike a real-time PCR which uses a standard curve to quantify the relative concentration of target DNA/RNA, according to the droplet digital PCR, it is possible to absolutely quantify the concentration of target DNA/RNA without using a standard curve. The droplet digital PCR is generally used as a validated method for determining the VAF because it can quantify the concentration of target DNA/RNA with high accuracy and high sensitivity.

### Step of introducing a mutated target sequence and an internal standard sequence into genomic DNA

The method of the present embodiment includes a step of introducing a mutated target sequence and an internal standard sequence into genomic DNA.

The target sequence is derived from genomic DNA.

The mutated target sequence and the internal standard sequence may be introduced into genomic DNA separately, but it is preferable that they be introduced into genomic DNA together. That is, the sequence to be introduced into genomic DNA is preferably a sequence containing both the mutated target sequence and the internal standard sequence. In addition, the introduced sequence may contain a selection marker in addition to the mutated target sequence and the internal standard sequence. When the selection marker is contained, the genomic DNA into which the mutated target sequence and the internal standard sequence are introduced can be efficiently selected and collected. Examples of selection markers include antibiotic resistance genes and genes that express fluorescent proteins.

The sites where the mutated target sequence and the internal standard sequence are introduced may be arbitrary, but are preferably different from the region of the target sequence in the genomic DNA or the region of the internal standard sequence that is the same as the internal standard sequence to be introduced.

The method of introducing a mutated target sequence and an internal standard sequence into genomic DNA may be appropriately determined by those skilled in the art, and introduction using a recombinant vector is preferable. That is, the mutated target sequence and the internal standard sequence are preferably derived from a recombinant vector.

As used herein, the term "recombinant vector" refers to a nucleic acid used for recombining a target gene, which carries the target gene to a gene sequence to be recombined. The recombinant vector is not particularly limited as long as it can be incorporated into genomic DNA and can achieve stable expression. Examples of types of recombinant vectors include non-viral vectors such as a transposon vector and a plasmid vector, and viral vectors. Among these, a transposon vector is preferable. In addition, the transposon vector is preferably, for example, a ToI2 transposon vector.

When the recombinant vector is a transposon vector, the mutated target sequence and the internal standard sequence are introduced into genomic DNA using a transposon system, when the recombinant vector is a viral vector, they are introduced by infecting a host with the viral vector, and when the recombinant vector is a non-viral vector, they are introduced by introducing the recombinant vector into a host by transformation. When the transposon system is used, a transposase vector expressing a transfer enzyme (transposase), mRNA or the like may be used together with a transposon vector having a mutated target sequence and an internal standard sequence.

The recombinant vector into which the mutated target sequence and the internal standard sequence are introduced may be produced by a method based on a nucleic acid amplification reaction such as a PCR, but is preferably produced by a method including a restriction enzyme treatment step and a ligation step without using a nucleic acid amplification reaction. For example, a recombinant vector having a mutated target sequence can be produced without using a nucleic acid amplification reaction by treating the recombinant vector with a restriction enzyme and inserting a double-stranded nucleic acid containing the mutated target sequence into the cleavage site and performing ligation. More specifically, for example, a recombinant vector can be produced by a method including 1) a step of cleaving a recombinant vector with a first restriction enzyme, wherein the recombinant vector has a nucleic acid sequence in which a first restriction enzyme recognition sequence, a second restriction enzyme recognition sequence, and a first restriction enzyme recognition sequence are arranged in that order, the two first restriction enzyme recognition sequences are arranged such that cleavage sites for two first restriction enzymes are located on the sides opposite to the second restriction enzyme recognition sequence, and the first restriction enzyme recognition sequence is a Type IIS restriction enzyme recognition sequence, and the second restriction enzyme recognition sequence is a restriction enzyme recognition sequence different from the first restriction enzyme recognition sequence, and 2) a step of ligating a double-stranded nucleic acid containing a region composed of a polymorphic nucleotide in a target sequence and its 5'-side and 3'-side flanking sequences together with their complementary sequences. The method will be described in detail below.

The method of producing a recombinant vector includes a step of cleaving, with a first restriction enzyme, a recombinant vector having a nucleic acid sequence in which a first restriction enzyme recognition sequence, a second restriction enzyme recognition sequence, and a first restriction enzyme recognition sequence are arranged in that order.

As used herein, the first restriction enzyme (which may be one or two) is a Type IIS restriction enzyme. The Type IIS restriction enzyme generally refers to a restriction enzyme that cleaves at a cleavage site separated from its restriction enzyme recognition sequence. Among Type IIS restriction enzymes, a Type IIS restriction enzyme that has all cleavage sites on one side separated from its restriction enzyme recognition sequence is preferable. In addition, the recognition sequence of the Type IIS restriction enzyme need not be a palindromic sequence. Examples of Type IIS restriction enzymes include Alwl, AlwXI, Alw26I, Bbsl, Bbvl, Bbvll, Bcefl, Bccl, Bcgl, BciVI, BinI, Bmrl, Bpml, Bsal, BseRl, Bsgl, BsmAl, BsmBI, BspMI, BsrDI, BstF5I, Earl, Eco31I, Eco57I, Esp3I, Esp3I, Faul, Fokl, Gsul, Hgal, HinGUII, Hphl, Ksp632I, Mboll, Mmel, Mn1I, NgoVIII, PaqCl, Plel, Psrl, RleAl, Sapl, SfaNl, Taqll, Tth111II, Acul, Bsml, Bsrl, BsmFl, BseMII, BspQl, and BtgZl. Among these, Bsml is preferable.

In addition, as used herein, in a nucleic acid sequence in which a first restriction enzyme recognition sequence, a second restriction enzyme recognition sequence, and a first restriction enzyme recognition sequence are arranged in that order, the two first restriction enzyme recognition sequences may be distinguished from each other: the former as a first restriction enzyme recognition sequence A, and the latter as a first restriction enzyme recognition sequence B. In addition, when viewed from either one of single-stranded sequences of the double-stranded sequence of the second restriction enzyme recognition sequence, one located on the 5'-side may be distinguished as a 5'-side first restriction enzyme recognition sequence, and one located on the 3'-side may be distinguished as a 3'-side first restriction enzyme recognition sequence.

The two first restriction enzyme recognition sequences may be restriction enzyme recognition sequences that the same first restriction enzymes recognize or restriction enzyme recognition sequences that different first restriction enzymes recognize.

When the two first restriction enzyme recognition sequences are restriction enzyme recognition sequences that the same first restriction enzymes recognize, the two first restriction enzyme recognition sequences may be the same sequence. When the two first restriction enzyme recognition sequences are restriction enzyme recognition sequences that the same first restriction enzymes recognize, and the first restriction enzyme is a restriction enzyme that recognizes a plurality of nucleic acid sequences, the two first restriction enzyme recognition sequences may be sequences different from each other.

In addition, when the two first restriction enzyme recognition sequences are restriction enzyme recognition sequences that different first restriction enzymes recognize, the two first restriction enzyme recognition sequences may be sequences different from each other. When the two first restriction enzyme recognition sequences are restriction enzyme recognition sequences that different first restriction enzymes recognize, and the different first restriction enzymes recognize the same sequence, the two first restriction enzyme recognition sequences may be the same sequence.

The two first restriction enzyme recognition sequences may be sequences different from each other as described above, but they are preferably the same sequence, and more preferably the same sequence that the same first restriction enzymes recognize.

As used herein, the second restriction enzyme refers to any restriction enzyme different from the first restriction enzyme, and it preferably has a cleavage site within a nucleic acid sequence in which a first restriction enzyme recognition sequence, a second restriction enzyme recognition sequence, and a first restriction enzyme recognition sequence are arranged in that order, and has only one cleavage site in the recombinant vector. In addition, it preferably has no cleavage site in a double-stranded nucleic acid containing a region composed of a polymorphic nucleotide in a target sequence and its 5'-side and 3'-side flanking sequences together with their complementary sequences, which is inserted in step 2). For example, although not particularly limited, Notl is preferable.

In the nucleic acid sequence in which a first restriction enzyme recognition sequence, a second restriction enzyme recognition sequence, and a first restriction enzyme recognition sequence are arranged in that order, the first restriction enzyme recognition sequences are preferably arranged at least 3 nucleotides apart and more preferably arranged at least 6 nucleotides apart.

In one embodiment, the number of nucleotides between the first restriction enzyme recognition sequences is selected from the group consisting of 3 nucleotides, 4 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides and 20 nucleotides.

The nucleic acid sequence in which a first restriction enzyme recognition sequence, a second restriction enzyme recognition sequence, and a first restriction enzyme recognition sequence are arranged in that order may contain a linker sequence having any number of nucleotides.

A first restriction enzyme recognition sequence, a linker, a second restriction enzyme recognition sequence, and a first restriction enzyme recognition sequence may be arranged in that order, a first restriction enzyme recognition sequence, a second restriction enzyme recognition sequence, a linker, and a first restriction enzyme recognition sequence may be arranged in that order, and a first restriction enzyme recognition sequence, a linker, a second restriction enzyme recognition sequence, a linker, and a first restriction enzyme recognition sequence may be arranged in that order.

In addition, a linker, a first restriction enzyme recognition sequence, a linker, a second restriction enzyme recognition sequence, and a first restriction enzyme recognition sequence may be arranged in that order, a linker, a first restriction enzyme recognition sequence, a second restriction enzyme recognition sequence, a linker, and a first restriction enzyme recognition sequence may be arranged in that order, and a linker, a first restriction enzyme recognition sequence, a linker, a second restriction enzyme recognition sequence, a linker, and a first restriction enzyme recognition sequence may be arranged in that order.

In addition, a first restriction enzyme recognition sequence, a linker, a second restriction enzyme recognition sequence, a first restriction enzyme recognition sequence, and a linker may be arranged in that order, a first restriction enzyme recognition sequence, a second restriction enzyme recognition sequence, a linker, a first restriction enzyme recognition sequence, and a linker may be arranged in that order, and a first restriction enzyme recognition sequence, a linker, a second restriction enzyme recognition sequence, a linker, a first restriction enzyme recognition sequence, and a linker may be arranged in that order.

In addition, a linker, a first restriction enzyme recognition sequence, a linker, a second restriction enzyme recognition sequence, a first restriction enzyme recognition sequence, and a linker may be arranged in that order, a linker, a first restriction enzyme recognition sequence, a second restriction enzyme recognition sequence, a linker, a first restriction enzyme recognition sequence, and a linker may be arranged in that order, and a linker, a first restriction enzyme recognition sequence, a linker, a second restriction enzyme recognition sequence, a linker, a first restriction enzyme recognition sequence, and a linker may be arranged in that order. The number of nucleotides constituting the linker sequence is not particularly limited as long as desired effects are obtained.

In one embodiment, the number of nucleotides constituting the linker sequence is selected from the group consisting of 1 nucleotide, 2 nucleotides, 3 nucleotides, 4 nucleotides, 5 nucleotides, 6 nucleotides, 7 nucleotides, 8 nucleotides, 9 nucleotides and 10 nucleotides.

In the nucleic acid sequence in which a first restriction enzyme recognition sequence, a second restriction enzyme recognition sequence, and a first restriction enzyme recognition sequence are arranged in that order, the two first restriction enzyme recognition sequences are arranged such that cleavage sites for two first restriction enzymes are located on the sides opposite to the second restriction enzyme recognition sequence. For example, when the first restriction enzyme recognition sequence is cleaved at sites 1 to 5 nucleotides apart from the recognition sequence, the first restriction enzyme recognition sequence and the second restriction enzyme recognition sequence are arranged such that cleavage sites indicated by underlines (the bond between two underlined nucleotides is cleaved) are represented as: 5'-NNNNNN-(first instance of first restriction enzyme recognition sequence)-(second restriction enzyme recognition sequence)-(second instance of first restriction enzyme recognition sequence)-NNNNNN-3', and in the complementary strand, 3'-NNNNNN- (first instance of first restriction enzyme recognition sequence)-(second restriction enzyme recognition sequence)-(second instance of first restriction enzyme recognition sequence)-NNNNNN-5'. Figure 1 is a schematic diagram showing the arrangement of the first restriction enzyme recognition sequence and the second restriction enzyme recognition sequence in the recombinant vector when the first restriction enzyme recognition sequence is cleaved at sites 1 to 5 nucleotides apart from the recognition sequence. In Figure 1, N represents any nucleotide, and is a part of the target gene sequence in the vicinity of the site where a mutation is to be inserted.

As described above, the recombinant vector containing a nucleic acid sequence in which a first restriction enzyme recognition sequence and a second restriction enzyme recognition sequence are arranged preferably has only two cleavage sites for the first restriction enzyme. That is, the recombinant vector preferably has only two first restriction enzyme recognition sequences.

As described above, a nucleic acid sequence in which a first restriction enzyme recognition sequence and a second restriction enzyme recognition sequence are arranged is cleaved with a first restriction enzyme. Reaction conditions such as the time for reaction with the first restriction enzyme and the treatment temperature can be appropriately determined by those skilled in the art based on the type of the first restriction enzyme.

When a recombinant vector having a nucleic acid sequence in which a first restriction enzyme recognition sequence, a second restriction enzyme recognition sequence, and a first restriction enzyme recognition sequence are arranged in that order is cleaved with a first restriction enzyme, in the recombinant vector, a site having a nucleic acid sequence in which a first restriction enzyme recognition sequence, a second restriction enzyme recognition sequence, and a first restriction enzyme recognition sequence are arranged in that order is excised.

The method of producing a recombinant vector further includes a step of ligating a double-stranded nucleic acid containing a region composed of a polymorphic nucleotide in a target sequence and its 5'-side and 3'-side flanking sequences together with their complementary sequences.

As used herein, the term "ligation" refers to a reaction in which nucleic acids are linked together via a phosphate diester bond using a ligase. The ligase used in the ligation step and reaction conditions such as the time for reaction with the ligase and the treatment temperature can be appropriately determined by those skilled in the art. Commercially available ligation reagents may be used.

The polymorphic nucleotide in a target sequence and its 5'-side and 3'-side flanking sequences, and their complementary sequences preferably have 10 or more and 200 or less nucleotide pairs.

In one embodiment, the polymorphic nucleotide in a target sequence and its 5'-side and 3'-side flanking sequences, and their complementary sequences have 10 or more and 150 or less nucleotide pairs and preferably have 15 or more and 100 or less nucleotide pairs.

The ligation step is preferably performed after the step of cleaving with the first restriction enzyme.

A double-stranded nucleic acid containing a region composed of a polymorphic nucleotide in a target sequence and its 5'-side and 3'-side flanking sequences together with their complementary sequences is inserted into a location where a site having a nucleic acid sequence in which a first restriction enzyme recognition sequence, a second restriction enzyme recognition sequence, and a first restriction enzyme recognition sequence are arranged in that order is excised according to the first restriction enzyme treatment, and ligated. Figure 2 is a schematic diagram showing a case in which the recombinant vector shown as an example in Figure 1 is treated with a first restriction enzyme recognition sequence that cleaves at sites 1 to 5 nucleotides apart from the recognition sequence, and a double-stranded nucleic acid is then inserted and ligated. In Figure 2, N represents any nucleotide, and is a part of the target gene sequence in the vicinity of the site where a mutation is to be inserted, and when the cleavage sequence with the first restriction enzyme has a 5' cohesive end or a 3' cohesive end, N in a region forming a double strand with a part of a 5'-side or 3'-side flanking sequence, and its complementary sequence is a part of a 5'-side or 3'-side flanking sequence and its complementary sequence.

When the cleavage sequence, from which a site having a nucleic acid sequence in which a first restriction enzyme recognition sequence, a second restriction enzyme recognition sequence, and a first restriction enzyme recognition sequence are arranged in that order is excised according to the first restriction enzyme treatment, has a 5' cohesive end or a 3' cohesive end, the double-stranded nucleic acid to be ligated preferably has a 3' cohesive end or a 5' cohesive end such that it forms a complementary strand with the 5' cohesive end or the 3' cohesive end. When the cleavage sequence generated in the recombinant vector according to the first restriction enzyme treatment has only blunt ends, the double-stranded nucleic acid to be ligated may be a nucleic acid having blunt ends.

The method of producing a recombinant vector may further include a step of annealing the polymorphic nucleotide in a target sequence and its 5'-side and 3'-side flanking sequences with their complementary sequences. The annealing step is preferably performed before the ligation step.

As used herein, annealing refers to a reaction in which a single-stranded nucleic acid and its complementary single-stranded nucleic acid form a double strand. Generally, a double-stranded nucleic acid is formed by treatment at a specific annealing temperature. The annealing temperature is appropriately set by those skilled in the art, and is determined based on, for example, a Tm value specific to the sequence. The annealing step is performed by treating a polymorphic nucleotide and its 5'-side and 3'-side flanking sequences, and their complementary sequences at an annealing temperature calculated based on these sequences and the like. The thermal denaturation treatment is performed to form single-stranded nucleic acids, which are then treated at an annealing temperature. The thermal denaturation treatment is performed, for example, at about 95°C. In addition, for example, annealing may be performed by stepwise cooling from the thermal denaturation treatment temperature.

According to the method of producing a recombinant vector, a site having a nucleic acid sequence in which a first restriction enzyme recognition sequence, a second restriction enzyme recognition sequence, and a first restriction enzyme recognition sequence are arranged in that order is excised from the recombinant vector, a double-stranded nucleic acid containing a polymorphic nucleotide is ligated thereto, and thus the recombinant vector containing the polymorphic nucleotide can be prepared.

The recombinant vector into which a double-stranded nucleic acid has been inserted also includes those in which, after the recombinant vector is cleaved with the first restriction enzyme, instead of a double-stranded nucleic acid containing a polymorphic nucleotide, a double-stranded nucleic acid containing a nucleic acid sequence in which a first restriction enzyme recognition sequence, a second restriction enzyme recognition sequence, and a first restriction enzyme recognition sequence are arranged in that order, which is excised, is inserted, and self-ligated. In addition, it also includes those that are not cleaved at all with the first restriction enzyme and those that are cleaved at only one site with the first restriction enzyme.

Therefore, when the recombinant vector is treated with a second restriction enzyme, it is possible to confirm whether the double-stranded nucleic acid containing a polymorphic nucleotide has been inserted into the recombinant vector.

When the mutated target sequence and the internal standard sequence are introduced into genomic DNA using a recombinant vector, for example, a cell containing genomic DNA is transformed using the recombinant vector, and thus the mutated target sequence and the internal standard sequence can be incorporated into the genomic DNA.

Transformation may be performed by any method known to those skilled in the art. Examples of transformation methods include an electroporation method and a heat shock method. In addition, transformation may also be performed using a commercially available kit.

After transformation, selection, growth, and collection of cells containing genomic DNA into which the mutated target sequence and the internal standard sequence are introduced may be performed by any method known to those skilled in the art. When a selection marker is introduced into genomic DNA together with the mutated target sequence and the internal standard sequence, for example, when an antibiotic resistance gene or a gene that expresses a fluorescent protein is introduced, cells containing genomic DNA into which the mutated target sequence and the internal standard sequence are introduced can be selected by adding an antibiotic or by performing observation under a fluorescent microscope.

It is preferable that the introduction of the mutated target sequence and the internal standard sequence into genomic DNA be finally confirmed by amplifying a region including the mutated target sequence and the internal standard sequence in the genomic DNA and subjecting the amplification product to sequence analysis.

Cells containing genomic DNA in which the introduction of the mutated target sequence and the internal standard sequence has been confirmed are collected, genomic DNA is extracted from the cells, and thus genomic DNA as a target calibration reference material can be obtained.

### Calculation of VAF of genomic DNA as calibration reference material

Since the genomic DNA as the calibration reference material produced by the method of the present embodiment contains a mutated target sequence, it can be widely used for analyzing a mutation in the target sequence. In order to analyze a mutation in the target sequence, for example, the copy number of the mutated target sequence in the genomic DNA as a calibration reference material can be used.

Herein, the internal standard sequence introduced into the genomic DNA together with the mutated target sequence is referred to as a first internal standard sequence.

Since the target sequence and the first internal standard sequence are derived from genomic DNA, the genomic DNA into which the mutated target sequence and the first internal standard sequence are introduced is genomic DNA containing a non-mutated wild-type target sequence and the first internal standard sequence.

Thus, when one copy of the mutated target sequence and one copy of the first internal standard sequence are introduced into genomic DNA containing one copy of the wild-type target sequence and one copy of the first internal standard sequence, the resulting genomic DNA contains one copy of the wild-type target sequence, one copy of the mutated target sequence, and two copies of the first internal standard sequence.

Therefore, in the genomic DNA as the calibration reference material produced by the method of the present embodiment, the copy number of the mutated target sequence is equal to the copy number of the first internal standard sequence, which is increased by being introduced together with the mutated target sequence. The increase in the copy number of the first internal standard sequence can be calculated by subtracting the copy number of the first internal standard sequence in the genomic DNA before gene introduction from the copy number of the first internal standard sequence in the genomic DNA after gene introduction (Formula 1). [Formula 1] 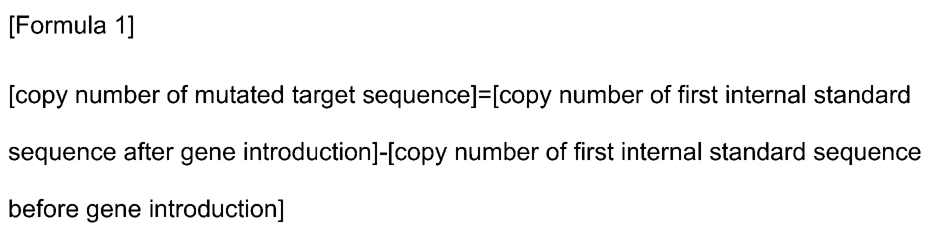 [copy number of mutated target sequence]=[copy number of first internal standard sequence after gene introduction]-[copy number of first internal standard sequence before gene introduction]

In addition, here, any internal standard sequence that is inherent to genomic DNA and different from the first internal standard sequence is referred to as a second internal standard sequence. The first internal standard sequence and the second internal standard sequence may be sequences derived from different gene loci or may be sequences derived from different chromosomes.

When the genomic DNA is derived from a cell in which the chromosome number is diploid, the copy number of the first internal standard sequence and the copy number of the second internal standard sequence are the same in the genomic DNA before the mutated target sequence and the internal standard sequence are introduced. Even after the mutated target sequence and the internal standard sequence are introduced, the copy number of the second internal standard sequence does not change (Formula 2). [Formula 2] 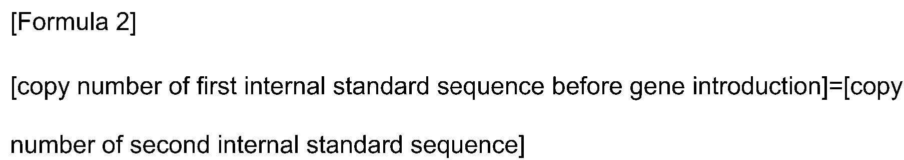 [copy number of first internal standard sequence before gene introduction]=[copy number of second internal standard sequence]

Therefore, from [Formula 1] and [Formula 2], the copy number of the mutated target sequence in the genomic DNA as the calibration reference material produced by the method of the present embodiment can be calculated by subtracting the copy number of the second internal standard sequence from the copy number of the first internal standard sequence in the genomic DNA after the mutated target sequence and the first internal standard sequence are introduced (Formula 3). [Formula 4] 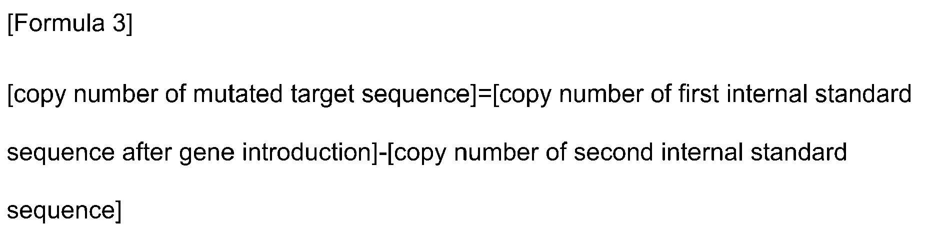 [copy number of mutated target sequence]=[copy number of first internal standard sequence after gene introduction]-[copy number of second internal standard sequence]

In addition, when the genomic DNA is derived from a cell in which the chromosome number is diploid, the copy number of the wild-type target sequence and the copy number of the second internal standard sequence are the same, and this relationship does not change even after the mutated target sequence and the internal standard sequence are introduced (Formula 4). [Formula 4] 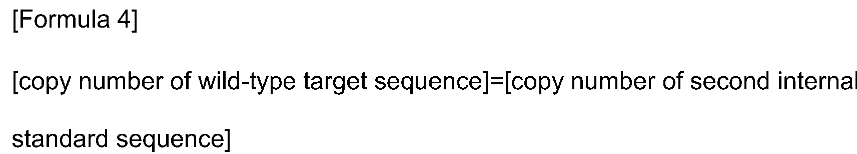 [copy number of wild-type target sequence]=[copy number of second internal standard sequence]

Furthermore, the VAF is generally calculated using the copy number of the mutated target sequence according to the calculation formula [Formula 5]. [Formula 5] $\begin{matrix}VAF \left(%\right) \\ = \left\{\left[copy number of mutated target sequence\right]/\left(\left[\begin{matrix}copy number of mutated target \\ sequence\end{matrix}\right]+\left[copy number of wild-type target sequence\right]\right)\right\} * 100\end{matrix}$

Therefore, from [Formula 3] to [Formula 5], the VAF of the genomic DNA as the calibration reference material can be calculated using the copy number of the first internal standard sequence and the copy number of the second internal standard sequence (Formula 6). [Formula 6] $\begin{matrix}VAF \left(%\right) \\ = \left\{\left(\left[copy number of first internal standard sequence after gene introduction\right]-\left[\begin{matrix}copy \\ number of second internal standard sequence\end{matrix}\right]\right)/\left(\left[\begin{matrix}copy number of first internal \\ standard sequence after gene introduction\end{matrix}\right]-\left[\begin{matrix}copy number of second internal \\ standard sequence\end{matrix}\right]+\left[copy number of second internal standard sequence\right]\right)\right\} * 100 \\ = \left\{\left(\left[copy number of first internal standard sequence after gene introduction\right]-\left[\begin{matrix}copy \\ number of second internal standard sequence\end{matrix}\right]\right)/\left[\begin{matrix}copy number of first internal \\ standard sequence after gene introduction\end{matrix}\right]\right\} * 100\end{matrix}$

The genomic DNA is preferably derived from a cell in which the chromosome number is diploid. However, when the chromosome number is not diploid, the VAF can be calculated in the same manner as described above by selecting the first internal standard sequence and the second internal standard sequence such that the first internal standard sequence, the second internal standard sequence, and the target sequence each have the same number of chromosomes.

Therefore, when the genomic DNA as the calibration reference material is produced by the method including a step of introducing a mutated target sequence and an internal standard sequence into genomic DNA according to the present embodiment, the VAF of the calibration reference material can be calculated using the copy numbers of two types of internal standard genes without using the copy number of the mutant gene. That is, by the method of the present embodiment, the VAF of the calibration reference material can be accurately calculated without preparing a validated droplet digital PCR reagent for each mutant gene. Since validated droplet digital PCR reagents for two types of internal standard genes can be commonly used for the calibration reference materials containing respective mutant genes, the VAFs of a plurality of calibration reference materials can be easily calculated.

The method of the present embodiment may be a method of producing genomic DNA as a calibration reference material by which the VAF can be easily calculated.

### Analysis of mutation in target sequence using genomic DNA as calibration reference material

The calibration reference material produced according to the method of the present embodiment and in which the VAF or the copy number of the mutant gene is defined can be used to evaluate the sensitivity of a companion diagnostic agent.

As used herein, the companion diagnostic agent is a diagnostic agent for examining the presence of an amino acid mutation in order to administer a molecularly targeted drug, which is a chemotherapeutic agent targeting an abnormal enzyme having the amino acid mutation.

Among companion diagnostic agents, for example, the sensitivity of a gene mutation diagnostic agent, and particularly, for example, the detection sensitivity of the mutant gene, is defined by the VAF or the copy number of the mutant gene, and is evaluated using a calibration reference material in which the VAF or the copy number of the mutant gene is defined.

As used herein, the detection sensitivity of the gene mutation diagnostic agent is a minimum frequency of a mutant gene that can be detected by the diagnostic agent in a sample to be examined by the diagnostic agent, and is defined using the VAF, which is an index of the frequency of the mutant gene. The detection sensitivity of the gene mutation diagnostic agent is a minimum amount of the mutant gene that can be detected by the diagnostic agent in a sample to be examined by the diagnostic agent, and in this case, it is defined using the copy number of the mutant gene. In addition, it may be defined by the copy number calculated from the VAF or may be defined by the VAF calculated from the copy number.

The detection sensitivity of the mutant gene is synonymous with minimum detection sensitivity, minimum detectable gene abnormality frequency, limit of detection or the like.

The detection sensitivity can be evaluated, for example, by detecting a mutation using a mutant gene diagnostic agent in genomic DNA as the calibration reference material in which the VAF is defined. In addition, for example, it can be evaluated by detecting a mutation using a mutant gene diagnostic agent in a sample containing a calibration reference material in which the copy number of the mutant gene is defined.

An example of a specific method of evaluating the detection sensitivity will be described below. For example, when evaluation is performed using a calibration reference material in which a VAF of 1% is defined, if a mutation is detected by examining a calibration reference material in which the VAF is defined with the gene mutation diagnostic agent whose detection sensitivity is to be evaluated, the detection sensitivity of the gene mutation diagnostic agent (VAF) can be evaluated as 1% or more. In addition, for example, when evaluation is performed using a calibration reference material in which the copy number of a mutant gene is defined as 30, if a mutation is detected by examining a sample containing a calibration reference material in which the copy number is defined with the gene mutation diagnostic agent whose detection sensitivity is to be evaluated, the detection sensitivity of the gene mutation diagnostic agent can be evaluated as 30 copies or more., and when the examined sample contains a total of 3000 copies of the gene, the detection sensitivity (VAF) can be evaluated as 1% or more.

The evaluation of the detection sensitivity can be used as an index for validation of a companion diagnostic agent such as a gene mutation diagnostic agent.

Therefore, the method of the present embodiment may be a method of producing genomic DNA as a calibration reference material used in companion diagnostics, a method of producing genomic DNA as a calibration reference material to be used in evaluating the sensitivity of a companion diagnostic agent, a method of producing genomic DNA as a calibration reference material to be used in evaluating the sensitivity of a gene mutation diagnostic agent, or a method of producing genomic DNA as a calibration reference material to be used for validation of a companion diagnostic agent.

In addition, the present embodiment also provides genomic DNA produced by a method including a step of introducing a mutated target sequence and an internal standard sequence into genomic DNA.

The method of producing genomic DNA as a calibration reference material according to the first embodiment also applies to second and third embodiments described below.

### (Cell containing genomic DNA into which mutated target sequence and internal standard sequence are introduced)

The second embodiment provides a cell containing one or more copies of genomic DNA into which a mutated target sequence and an internal standard sequence have been introduced.

The copy number of the genomic DNA into which the mutated target sequence and the internal standard sequence are introduced, which is contained in the cell of the present embodiment, can be appropriately determined by those skilled in the art according to the purpose.

In addition, the cell provided in the present embodiment can be used as a calibration reference material, and tissues containing cells as a calibration reference material can also be used as a calibration reference material. Therefore, the present embodiment also provides cells as a calibration reference material and tissues as a calibration reference material. Examples of tissues as calibration reference materials include a formalin-fixed paraffin-embedded (FFPE) tissue sample.

The cell of the present embodiment may be used to produce genomic DNA as a calibration reference material.

When the cell of the present embodiment is used as a calibration reference material, the chromosome number in the cell is preferably diploid.

The VAF of the calibration reference material having each mutation derived from the cell of the present embodiment can be accurately calculated using the copy number of the internal standard sequence without using the copy number of each mutation sequence. In addition, since the validated droplet digital PCR reagent for the internal standard gene can be commonly used for a calibration reference material containing each mutant gene without preparing a validated droplet digital PCR reagent for each mutant gene, the VAFs of a plurality of calibration reference materials can be easily calculated.

The cell of the present embodiment may be a cell used in companion diagnostics, a cell used in evaluating the sensitivity of a companion diagnostic agent, or a cell used in evaluating the sensitivity of a gene mutation diagnostic agent. It may be a cell used for validation of a companion diagnostic agent.

### (Method of analyzing mutation in target sequence)

A third embodiment provides a method of analyzing a mutation in a target sequence, including 1) a step of subjecting genomic DNA into which a mutated target sequence and an internal standard sequence have been introduced to an amplification reaction, and 2) a step of calculating the copy numbers of the introduced internal standard sequence and a different internal standard sequence inherent to the genomic DNA.

### Amplification reaction step

The method of the present embodiment includes a step of subjecting genomic DNA into which a mutated target sequence and an internal standard sequence have been introduced to an amplification reaction. The amplification reaction may be performed by any method known to those skilled in the art, and is preferably performed by a PCR method, and particularly, more preferably performed by a droplet digital PCR method.

The conditions for each stage in the amplification reaction such as the temperature setting, the reaction time, and reagents to be used can be appropriately determined by those skilled in the art. When the amplification reaction is performed by a droplet digital PCR method, it is preferable to use a validated droplet digital PCR reagent including a probe validated for each gene to be amplified.

In the next step, it is preferable to amplify a region containing the first internal standard sequence and a region containing the second internal standard sequence so that the copy numbers of the first internal standard sequence introduced together with the mutated target sequence and the second internal standard sequence, which is any internal standard sequence inherent to genomic DNA and different from the first internal standard sequence can be calculated. In addition, regions containing any sequence other than the first internal standard sequence and the second internal standard sequence may be amplified together as long as the copy numbers of the first internal standard sequence and the second internal standard sequence can be calculated.

It is preferable to use separate primers for the amplification of the region containing the first internal standard sequence and the amplification of the region containing the second internal standard sequence.

Since the first internal standard sequence is derived from genomic DNA, for the amplification of the region containing the first internal standard sequence, it is preferable to amplify both the region containing the first internal standard sequence introduced together with the mutated target sequence and the region containing the first internal standard sequence inherent to the genomic DNA.

### Step of calculating copy number

The method of the present embodiment further includes a step of calculating the copy numbers of the introduced first internal standard sequence and the different second internal standard sequence inherent to the genomic DNA. The copy number can be calculated by quantitative analysis in the amplification reaction step.

The copy number of the mutated target sequence in the genomic DNA can be calculated using the copy number of the first internal standard sequence and the copy number of the second internal standard sequence, and additionally, the VAF of the genomic DNA can also be calculated using the copy number of the first internal standard sequence and the copy number of the second internal standard sequence. Therefore, the method of the present embodiment may further include a step of calculating the VAF.

### Step of calculating VAF

For example, the method further includes a step of calculating the VAF using the following [Formula 7]. The percentage value calculated from [Formula 7] can be used as the VAF of the genomic DNA. [Formula 7] 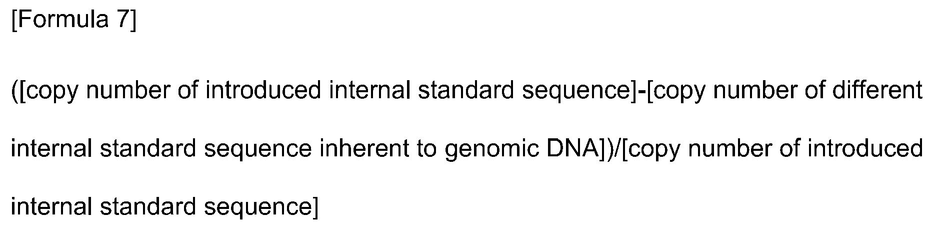 ([copy number of introduced internal standard sequence]-[copy number of different internal standard sequence inherent to genomic DNA])/[copy number of introduced internal standard sequence]

The above [Formula 7] can also be expressed as the following [Formula 8]. [Formula 8] 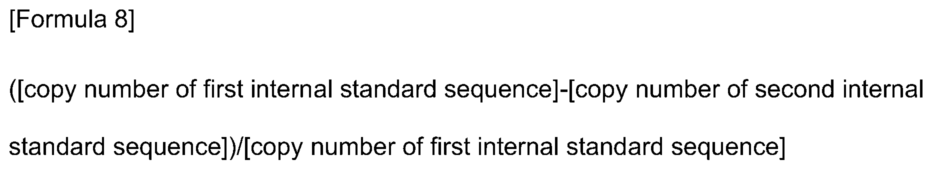 ([copy number of first internal standard sequence]-[copy number of second internal standard sequence])/[copy number of first internal standard sequence]

In addition, the values calculated from the above [Formula 7] and [Formula 8] are the same as or equivalent to the value calculated from the following [Formula 9], which is generally used to calculate the VAF. [Formula 9] 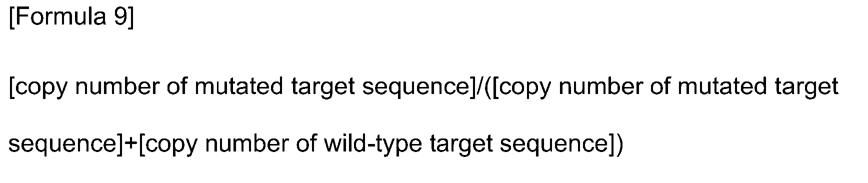 [copy number of mutated target sequence]/([copy number of mutated target sequence]+[copy number of wild-type target sequence])

According to the method of the present embodiment, since the copy number of the mutated target sequence and the VAF in the genomic DNA can be calculated, the method of the present embodiment can widely analyze mutations in the target sequence in the genomic DNA.

When the genomic DNA is a calibration reference material, the VAF calculated by the method of the present embodiment can be used to evaluate the sensitivity of a reagent for detecting a mutation, for example, a companion diagnostic agent such as a gene mutation diagnostic agent.

Therefore, the method of the present embodiment may be a method of analyzing a mutation in a target sequence used in companion diagnostics, a method of analyzing a mutation in a target sequence used in evaluating the sensitivity of a companion diagnostic agent, a method of analyzing a mutation in a target sequence to be used in evaluating the sensitivity of a gene mutation diagnostic agent, or a method of analyzing a mutation in a target sequence to be used for validation of a companion diagnostic agent.

### [Examples]

### Example 1

### <HEL299 cell culture>

HEL299 cells, which are normal diploid (https://www.atcc.org/products/ccl-137) and do not have mutations in major oncogenes (https://www.cellosaurus.org/CVCL_2480), were obtained from ATCC and used. HEL299 cells stored in a freezer at -150°C were rapidly lysed in a water bath at 37°C, and suspended in a complete medium (EMEM supplemented with 10% fetal bovine serum (Cat: 051-07615, commercially available from FUJIFILM Wako Pure Chemical Corporation) heated at 37°C in advance, and subjected to a centrifugation operation (130 x g, 5 min, room temperature), the resulting pellets were then suspended again in a new complete medium, and then seeded into a T-25 flask, and culturing was initiated in a CO₂ incubator under conditions of 37°C, 5% CO₂, and water vapor saturation. When the cells reached subconfluence, the culture supernatant was removed, the culture surface was washed with an appropriate amount of D-PBS (-) heated to 37°C in advance (commercially available from FUJIFILM Wako Pure Chemical Corporation, Cat: 045-29795), a Trypsin/EDTA solution (commercially available from FUJIFILM Wako Pure Chemical Corporation, Cat: 201-16945) was added, the mixture was left in a CO₂ incubator, and the cells were detached. A volume of a complete medium heated to 37°C in advance, at least five times the volume of Trypsin/EDTA, was added to the detached cells, and the cell suspension was collected in a conical tube (hereinafter referred to as PET treatment). After a centrifugation operation (130 x g, 5 min, room temperature), the resulting pellets were suspended again in a new complete medium. Then, the number of cells and the viability were evaluated using Trypan blue staining, the cells were then seeded into a new flask, and culturing was initiated in a CO₂ incubator under conditions of 37°C, 5% CO₂, and water vapor saturation.

### <HEL299 gene introduction>

Cells cultured for three passages after cell lysis were seeded in a 6-well multi plate at 600,000 cells/well, and cultured for about 24 hours. After confirming that the cells were 80-90% confluent, and gene introduction was performed using a Lipofectamine 3000 kit (Thermo Fisher Scientific L, Cat: 3000-008) according to the kit's instructions. The gene introduction conditions were: the amount of Lipofectamine was 5.0 µL/well, the amount of introduced DNA was 1.25 µg/well, and the ratio of ToI2 pDNA (SEQ ID NO: 1 or 2)/ToI2 Transposase pDNA (SEQ ID NO: 3) was 2:1. Figure 3 shows an example of ToI2 pDNA (SEQ ID NO: 1). After the cell culture supernatant was removed, 1,000 µL/well of a complete medium heated to 37°C in advance was added. In a sterilized microtube, each dose of Lipofectamine 3000 was added to 125 µL OptiMEM, mixed by vortexing for 3 seconds and then spun down (Solution 1). In another microtube, 125 µL of OptiMEM (gibco, Cat: 31985-062), each dose of pDNA, and 2 µL of a P3000 reagent per 1 µg of pDNA were added, and mixed by tapping, Solution 1 was then added, and additionally mixed by tapping, and the mixture was then left at room temperature for 15 minutes. Then, the Lipofectamine:pDNA complex was added dropwise to the cells using Dropwise, mixed well, and then returned to the CO₂ incubator. After 2 hours or longer had elapsed, 1,000 µL/well of a complete medium heated to 37°C in advance was added, and culturing was performed overnight.

### <Maintenance culture of transgene-stable recombinant HEL299 cells>

Cells for which 20 hours or longer had elapsed since gene introduction were observed under a microscope and under a fluorescent microscope (ToI2 pDNA contained EGFP and the introduced cells became GFP-positive) to confirm that the gene introduction had been achieved, the culture supernatant was removed, 2,000 µL/well of a complete medium heated to 37°C in advance was added. After additional culturing overnight, the medium was replaced with a complete medium supplemented with 0.4 µg/mL of Puromycin, and culturing was performed for an additional 4 days. The complete medium containing Puromycin was removed, a new complete medium was added, and culturing was performed until the cells again reached confluence. When the cells reached subconfluence, the medium was replaced again with a complete medium supplemented with 0.4 µg/mL of Puromycin, and culturing was performed for an additional 3 days. The complete medium containing Puromycin was removed, a new complete medium was added, and culturing was performed until the cells reached confluence again. When the cells reached subconfluence, the cells were collected by a PET treatment and scale-up cultured in a T-75 flask. From the following day, the medium was replaced with a complete medium supplemented with 0.4 µg/mL of Puromycin, drug selection was applied for 3 days, and it was confirmed that the GFP positive rate was 100%. Thereafter, when the cells reached subconfluence, passaging operations were performed up to P+4, and the remaining cells in this process were collected, suspended in Cell Banker (TaKaRa, Cat: CB011) at 1,000,000 cells/mL, dispensed into cryotubes at 1 mL/tube, and cryopreserved.

### <gDNA extraction from HEL299 cells>

After HEL299 cells in which a transgene was stably incorporated or non-gene-introduced HEL299 cells as parent cells were collected, they were washed twice with D-PBS and cryopreserved as cell pellets. From the cryopreserved cell pellets, gDNA was purified using a QIAamp DNA Mini QlAcube Kit (QIAGEN, Cat: C 51326) or a Blood & Cell Culture DNA Mini Kit (QIAGEN, Cat: 13323). The gDNA concentration was determined using a Quant-iT dsDNA HS Assay Kit (Thermo Fisher Scientific, Cat: Q32854).

### <PCR amplification of integrated sequence>

In order to confirm that the sequence between "left end of ToI2" and "right end of ToI2" within ToI2 pDNA shown in Figure 3 was introduced into HEL299 cells, 10 ng of gDNA derived from HEL299 cells in which a transgene was stably incorporated was used as a template, a 300 nM ToI2 Seq PTEN primer (SEQ ID NO: 4), a 300 nM ToI2 Seq CMV primer (SEQ ID NO: 5), and a 0.5X KOD One PCR Master Mix (TOYOBO, Cat: KMM-101) were used as a reaction solution, a PCR reaction was performed for 30 cycles (one cycle: 98°C for 10 seconds, 55°C for 5 seconds, and 68°C for 5 seconds), and the reaction product was separated by electrophoresis on a 1.0% agarose/TAE gel and stained with an SYBR Gold staining reagent for 10 minutes to detect nucleic acids. When a target single band was detected, the DNA sequence of the product was determined by a direct sequencing method.

### <PCR product direct sequencing>

The concentration of the PCR product of the integrated sequence was determined using a Qubit dsDNA BR Assay kit (Thermo Fisher Scientific, Cat: Q32853), 10 fg of the product treated with an ExoSAP-IT Exp reagent (applied biosystems, Cat: 75001.200.UL) was used as a template, and sequence analysis was performed at Eurofins Genomics using each of two types of primers (SEQ ID NOs: 4 and 5) used in the PCR of the integrated sequence.

### <Measurement of copy number by ddPCR>

Using gDNA derived from HEL299 cells in which a transgene was stably incorporated or non-gene-introduced HEL299 cells as parent cells, the copy numbers of the PTEN gene (ddPCR Copy Number Assay: PTEN (FAM), Bio-Rad, Cat: 10031240), the BRAF WT gene and the BRAF V600E1 gene (ddPCR Mut assay, VAL BRAF V600E, Bio-Rad, Cat: 1080337), the KRAS WT gene and the KRAS G12A gene (ddPCR Mut assay, KRAS p. G12A, Human, Bio-Rad, Cat: 10049550) and the reference gene (AGO1 gene (ddPCR Copy Number Assay: AGO1 (HEX), Bio-Rad, Cat: 10031243), the ERBB2 gene (ddPCR CNV assay, ERBB2, Bio-Rad, Cat: 10031240), the TMEM11 gene (ddPCR, CNV assay, TMEM11, Bio-Rad, Cat: 10031243)) contained therein were measured by ddPCR according to the Bio-Rad recommended protocol. Based on the copy numbers of these genes per genome amount, the transgene copy number per cell and the Variant Allele Frequency (hereinafter referred to as VAF) were calculated according to the following formula.$\begin{matrix}Average copy number of transgene per cell \\ = \left\{\left(\left[PTEN gene copy number\right]-\left[reference gene copy number\right]\right)/\left[\begin{matrix}reference gene copy \\ number\end{matrix}\right]\right\} * 2\end{matrix}$
or $\left(\left[mutant gene copy number\right]/\left[wild-type gene copy number\right]\right) * 2$$\begin{matrix}VAF \left(%\right) \\ = \left\{\left(\left[PTEN gene copy number\right]-\left[reference gene copy number\right]\right)/\left[\begin{matrix}PTEN gene copy \\ number\end{matrix}\right]\right\} * 100\end{matrix}$
or 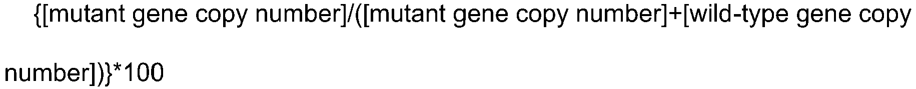 {[mutant gene copy number]/([mutant gene copy number]+[wild-type gene copy number])}*100

### Example 2

### <Result 1>

In the present invention, the copy number of a sequence incorporated into genomic DNA was determined by simultaneously measuring the copy numbers of an arbitrary first internal standard gene designed in the sequence incorporated into the genomic DNA (in this example, a sequence within the PTEN gene serving as a detection target for the ddPCR method was used) and an arbitrary second internal standard gene that was different from the first internal standard gene or genes to be incorporated into genomic DNA such as the BRAF mutant gene and the KRAS mutant gene.

In this example, in order to confirm the arbitrariness and robustness of the second internal standard gene, analysis was performed to confirm that the copy numbers of the AGO1 gene, the ERBB2 gene, and the TMEM11 gene did not change between HEL299 cells without gene introduction and HEL299 cells in which a transgene was stably incorporated.

Figure 4 shows the results of calculating the copy numbers of respective second internal standard genes using HEL299 cells without gene introduction (pink circle) and three types of cell lines in which a transgene was stably incorporated into the ToI2 system. As can be clearly understood from the results, when the genes were plotted on the X axis/Y axis, the relationship was X≈Y, and the copy number of the second internal standard gene per cell was considered to be constant regardless of which internal standard gene was measured.

### Example 3

### <Result 2>

Using HEL299 cells in which a human BRAF V600E1 gene partial sequence was stably incorporated (BRAF V600E1/HEL299), the BRAF WT gene copy number, the BRAF V600E1 gene copy number, the PTEN gene copy number, and the TMEM11 gene copy number were calculated. The samples used in the test were four types of independently established BRAF V600E1/HEL299 cells.

The validated ddPCR reagents used were BRAF wild-type (WT), BRAF V600E1, PTEN, and TMEM11.

As a result of determining the copy number by the ddPCR method, since the relationship between the gene copy numbers in the plot diagram was X≈Y, it was confirmed that BRAF WT gene copy number=TMEM11 gene copy number (Figure 5, the left panel), and BRAF V600E1 gene copy number=[PTEN gene copy number-TMEM11 gene copy number] (Figure 5, the center panel). Since the relationship between the VAF calculated from the BRAF WT gene copy number and the BRAF V600E1 gene copy number and the VAF calculated from the first internal standard gene (PTEN gene) copy number and the second internal standard gene (TMEM11 gene) copy number was also X≈Y, it was confirmed that the accuracy of the VAF calculated according to the present invention was high (Figure 5, the right panel).

### Example 4

### <Result 3>

Using HEL299 cells in which a human KRAS G12A gene partial sequence was stably incorporated (KRAS G12A/HEL299), the KRAS WT gene copy number, the KRAS G12A gene copy number, the PTEN gene copy number, and the AGO1 gene copy number were calculated. The samples used in the test were five types of independently established KRAS G12A/HEL299 cells.

The validated ddPCR reagents used were KRAS WT, KRAS G12A, PTEN, and AGO1.

As a result of determining the copy number by the ddPCR method, since the relationship between the gene copy numbers in the plot diagram was X≈Y, it was confirmed that KRAS WT gene copy number=AGO1 gene copy number (Figure 6, the left panel), and KRAS G12A gene copy number=[PTEN gene copy number-AGO1 gene copy number] (Figure 6, the center panel). Since the relationship between the VAF calculated from the KRAS WT gene copy number and the KRAS G12A gene copy number and the VAF calculated from the first internal standard gene (PTEN gene) copy number and the second internal standard gene (AGO1 gene) copy number was also X≈Y, it was confirmed that the accuracy of the VAF calculated according to the present invention was high (Figure 6, the right panel).

Table 1 shows the nucleic acid sequences used in this example.

**[Table 1]**

| SEQ ID NO | Name | Sequence (5' → 3' ) |
|---|---|---|
| 1 | BRAF V600E1-Toil pDNA | cagaggtgtaaagtacttgagtaattttacttgattactgtacttaagtattatttttggggatttttact ttacttgagtacaattaaaaatcaatacttttacttttacttaattacatttttttagaaaaaaagtact ttttactccttacaattttatttacagtcaaaaagtacttatttttggagatcacttcattctattttcc cttgctattaccaaaccaattgaattgcgctgatgcccagtttaatttaaatagatctcaactttgtatag aaaagttgcaaaaggagatatcaagaggatggattcgacttagacttgacctgtatccatttctgcggctg ctcctctttacctttctgtcactctcttagaacgtgggagtagacggatgcgaaaatgtccgtacggaaag catctcacctcatcctaacacatttcaagccccaaaaatcttaaaagcaggttatataggctaaatagaac taatcattgttttagacatacttattgactctaagaggaaagatgaagtactatgtttaaagaatattat attacagaattatagaaattagatctcttacctaaactcttcataatgcttgctctgataggaaaatgaga tctactgttttcctttacttactacacctcagatatatttcttcatgaagacctcacagtaaaaataggtg attttggtctagctacagagaaatctcgatggagtgggtcccatcagtttgaacagttgtctggatccatt ttgtggatggtaagaattgaggctattttcccactgattaaattttggccctgagatgctgctgagttac tagaaagtcattgaaggtctcaactatagtattttcatagttcccagtattcacaaaaatcagtgttctta tttttatgtaaatagattttttaactttttctttacccttaaaacgaatattttgaaaccagtttcagt gtatttcaaacaaaaatatatgtcttataaacagtgtttcatattggccggcctagttattaatagtaat caattacggggtcattagttcatagcccatatatggagttccgcgttacataacttacggtaaatggcccg cctggctgaccgcccaacgacccccgcccattgacgtcaataatgacgtatgttcccatagtaacgccaat agggactttccattgacgtcaatgggtggagtatttacggtaaactgcccacttggcagtacatcaagtgt atcatatgccaagtacgccccctattgacgtcaatgacggtaaatggcccgcctggcattatgcccagtac atgaccttatgggactttcctacttggcagtacatctacgtattagtcatcgctattaccatggtgatgcg gttttggcagtacatcaatgggcgtggatagcggtttgactcacggggatttccaagtctccaccccattg acgtcaatgggagtttgttttggcaccaaaatcaacgggactttccaaaatgtcgtaacaactccgcccca ttgacgcaaatgggcggtaggcgtgtacggtgggaggtctatataagcagagctggtttagtgaaccgtca gatccaagtttgtacaaaaaagcaggctgccaccatggtgagcaagggcgaggagctgttcaccggggtgg tgcccatcctggtcgagctggacggcgacgtaaacggccacaagttcagcgtgtccggcgagggcgagggc gatgccacctacggcaagctgaccctgaagttcatctgcaccaccggcaagctgcccgtgccctggcccac cctcgtgaccaccctgacctacggcgtgcagtgcttcagccgctaccccgaccacatgaagcagcacgact tcttcaagtccgccatgcccgaaggctacgtccaggagcgcaccatcttcttcaaggacgacggcaactac aagacccgcgccgaggtgaagttcgagggcgacaccctggtgaaccgcatcgagctgaagggcatcgactt caaggaggacggcaacatcctggggcacaagctggagtacaactacaacagccacaacgtctatatcatgg ccgacaagcagaagaacggcatcaaggtgaacttcaagatccgccacaacatcgaggacggcagcgtgcag ctcgccgaccactaccagcagaacacccccatcggcgacggccccgtgctgctgcccgacaaccactacct gagcacccagtccgccctgagcaaagaccccaacgagaagcgcgatcacatggtcctgctggagttcgtga ccgccgccgggatcactctcggcatggacgagctgtacaagatgggaagcggagagggcaggggaagtctt ctaacatgcggggacgtggaggaaaatcccggccccatgaccgagtacaagcccacggtgcgcctcgccac ccgcgacgacgtccccagggccgtacgcaccctcgccgccgcgttcgccgactaccccgccacgcgccaca ccgtcgatccggaccgccacatcgagcgggtcaccgagctgcaagaactcttcctcacgcgcgtcgggctc gacatcggcaaggtgtgggtcgcggacgacggcgccgcggtggcggtctggaccacgccggagagcgtcga agcgggggcggtgttcgccgagatcggcccgcgcatggccgagttgagcggttcccggctggccgcgcagc aacagatggaaggcctcctggcgccgcaccggcccaaggagcccgcgtggttcctggccaccgtcggagta tcgcccgaccaccagggcaagggtctgggcagcgccgtcgtgctccccggagtggaggcggccgagcgcgc cggggtgcccgccttcctggagacctccgcgccccgcaacctccccttctacgagcggctcggcttcaccg tcaccgccgacgtcgaggtgcccgaaggaccgcgcacctggtgcatgacccgcaagcccggtgcctgaacc cagctttcttgtacaaagtggtgatggccggccgcttcgagcagacatgataagatacattgatgagtttg gacaaaccacaactagaatgcagtgaaaaaaatgctttatttgtgaaatttgtgatgctattgctttattt gtaaccattataagctgcaataaacaagttaacaacaacaattgcattcattttatgtttcaggttcaggg ggaggtgtgggaggttttttaaagcaagtaaaacctctacaaatgtggtagatatcaagcttaaacaagaa tctctagttttctttcttgcttttactttacttccttaatactcaagtacaattttaatggagtactttt ttacttttactcaagtaagattctagccagatacttttacttttaattgagtaaaattttccctaagtact tgtactttcacttgagtaaaatttttgagtacttttacacctctgggcgctcttccgcttcctcgctcac tgactcgctgcgctcggtcgttcggctgcggcgagcggtatcagctcactcaaaggcggtaatacggttat ccacagaatcaggggataacgcaggaaagaacatgtgagcaaaaggccagcaaaaggccaggaaccgtaaa aaggccgcgttgctggcgtttttccataggctccgcccccctgacgagcatcacaaaaatcgacgctcaag tcagaggtggcgaaacccgacaggactataaagataccaggcgtttccccctggaagctccctcgtgcgct |
| | | ctcctgttccgaccctgccgcttaccggatacctgtccgcctttctcccttcgggaagcgtggcgctttct catagctcacgctgtaggtatctcagttcggtgtaggtcgttcgctccaagctgggctgtgtgcacgaacc ccccgttcagcccgaccgctgcgccttatccggtaactatcgtcttgagtccaacccggtaagacacgact tatcgccactggcagcagccactggtaacaggattagcagagcgaggtatgtaggcggtgctacagagttc ttgaagtggtggcctaactacggctacactagaagaacagtatttggtatctgcgctctgctgaagccagt taccttcggaaaaagagttggtagctcttgatccggcaaacaaaccaccgctggtagcggtggtttttttg tttgcaagcagcagattacgcgcagaaaaaaaggatctcaagaagatcctttgatcttttctacggggtct gacgctcagtggaacgaaaactcacgttaagggattttggtcatgagattatcaaaaaggatcttcaccta gatccttttaaattaaaaatgaagttttaaatcaatctaaagtatatatgagtaaacttggtctgacagtt accaatgcttaatcagtgaggcacctatctcagcgatctgtctatttcgttcatccatagttgcctgactc cccgtcgtgtagataactacgatacgggagggcttaccatctggccccagtgctgcaatgataccgcgaga cccacgctcaccggctccagatttatcagcaataaaccagccagccggaagggccgagcgcagaagtggtc ctgcaactttatccgcctccatccagtctattaattgttgccgggaagctagagtaagtagttcgccagtt aatagtttgcgcaacgttgttgccattgctacaggcatcgtggtgtcacgctcgtcgtttggtatggcttc attcagctccggttcccaacgatcaaggcgagttacatgatcccccatgttgtgcaaaaaagcggttagct ccttcggtcctccgatcgttgtcagaagtaagttggccgcagtgttatcactcatggttatggcagcactg cataattctcttactgtcatgccatccgtaagatgcttttctgtgactggtgagtactcaaccaagtcatt ctgagaatagtgtatgcggcgaccgagttgctcttgcccggcgtcaatacgggataataccgcgccacata gcagaactttaaaagtgctcatcattggaaaacgttcttcggggcgaaaactctcaaggatcttaccgctg ttgagatccagttcgatgtaacccactcgtgcacccaactgatcttcagcatcttttactttcaccagcgt ttctgggtgagcaaaaacaggaaggcaaaatgccgcaaaaaagggaataagggcgacacggaaatgttgaa tactcatactcttcctttttcaatattattgaagcatttatcagggttattgtctcatgagcggatacata tttgaatgtatttagaaaaataaacaaataggggttccgcgcacatttccccgaaaagtgccacctgacgt ctaagaaaccattattatcatgacattaacctataaaaataggcgtatcacgaggccctttcgtc |
| 2 | KRAS G12A-Tol2 pDNA | cagaggtgtaaagtacttgagtaattttacttgattactgtacttaagtattatttttggggatttttact ttacttgagtacaattaaaaatcaatacttttacttttacttaattacatttttttagaaaaaaagtact ttttactccttacaatttatttacagtcaaaaagtacttatttttggagatcacttcattctattttcc cttgctattaccaaaccaattgaattgcgctgatgcccagtttaatttaaatagatctcaactttgtatag aaaagttgcaaaaggagatatcaagaggatggattcgacttagacttgacctgtatccatttctgcggctg ctcctctttacctttctgtcactctcttagaacgtgggagtagacggatgcgaaaatgtccgtacggtaag tttatctcatgcatagtgttcggctttgggctgtggaatgttcaggcgtttcactgatgccagaaatggag cagaatctatcagctggagacaaaggccttgggcgggggtccttccatttggtgcctacgtggggagatct ttggagacagaagggagaatgggaaggagttgcggcctggaggcttcctgctagagctgagaagccttcgg ggagtaataggaaggggggatttccattgcttaggctgagggcggggcccaaggactgttgaaaaatagcta aggatgggggttgctagaaaactactccagaagtgtgaggccgatattaatccggtgtttttgcgttctct agtcactttaagaaccaaatggaaggtcacactagggttttcatttccattgattatagaaagctttaaag tactgtagatgtggctcgccaattaaccctgattactggtttccaacaggttcttgctggtgtgaaatgac tgagtacaaactggtggtggttggagcagctggtgttgggaaaagcgcactgacaatccagctaatccaga accactttgtagatgaatatgatcccaccatagaggtgaggcccagtggtagcccgctgacctgatcctgt ctctcacttgtcggatcatcttacccatattctgtattaaaggaataagaggagagaaagtaaaaagtta ttttgggtatacattcagttatgcaataagcttaacgtgtttatagagaacagttcatttttattagctgc tgaagtttctaaaacctgtccagttttaacagttctgtaaactattgcaaactcagtgttgagttcattc atgagtttcttcatatataacagctctattacatgagaaacacaggccatagtagcgagactgtctgattg tatgggagataataggatggagataaaggattcagagatgagtgttcttcaatatttatttattagctagt tgaagcagctgagaccagatgattggagtagcaagaacttgagatttttagtctttatgcctaggattttg gtccctgtttgcagtttatttagttgtgtgatattgagggccggcctagttattaatagtaatcaattacg gggtcattagttcatagcccatatatggagttccgcgttacataacttacggtaaatggcccgcctggctg accgcccaacgacccccgcccattgacgtcaataatgacgtatgttcccatagtaacgccaatagggactt tccattgacgtcaatgggtggagtatttacggtaaactgcccacttggcagtacatcaagtgtatcatatg ccaagtacgccccctattgacgtcaatgacggtaaatggcccgcctggcattatgcccagtacatgacctt atgggactttcctacttggcagtacatctacgtattagtcatcgctattaccatggtgatgcggttttggc agtacatcaatgggcgtggatagcggtttgactcacggggatttccaagtctccaccccattgacgtcaat gggagtttgttttggcaccaaaatcaacgggactttccaaaatgtcgtaacaactccgccccattgacgca aatgggcggtaggcgtgtacggtgggaggtctatataagcagagctggtttagtgaaccgtcagatccaag tttgtacaaaaaagcaggctgccaccatggtgagcaagggcgaggagctgttcaccggggtggtgcccatc |
| | | ctggtcgagctggacggcgacgtaaacggccacaagttcagcgtgtccggcgagggcgagggcgatgccac ctacggcaagctgaccctgaagttcatctgcaccaccggcaagctgcccgtgccctggcccaccctcgtga ccaccctgacctacggcgtgcagtgcttcagccgctaccccgaccacatgaagcagcacgacttcttcaag tccgccatgcccgaaggctacgtccaggagcgcaccatcttcttcaaggacgacggcaactacaagacccg cgccgaggtgaagttcgagggcgacaccctggtgaaccgcatcgagctgaagggcatcgacttcaaggagg acggcaacatcctggggcacaagctggagtacaactacaacagccacaacgtctatatcatggccgacaag cagaagaacggcatcaaggtgaacttcaagatccgccacaacatcgaggacggcagcgtgcagctcgccga ccactaccagcagaacacccccatcggcgacggccccgtgctgctgcccgacaaccactacctgagcaccc agtccgccctgagcaaagaccccaacgagaagcgcgatcacatggtcctgctggagttcgtgaccgccgcc gggatcactctcggcatggacgagctgtacaagatgggaagcggagagggcaggggaagtcttctaacatg cggggacgtggaggaaaatcccggccccatgaccgagtacaagcccacggtgcgcctcgccacccgcgacg acgtccccagggccgtacgcaccctcgccgccgcgttcgccgactaccccgccacgcgccacaccgtcgat ccggaccgccacatcgagcgggtcaccgagctgcaagaactcttcctcacgcgcgtcgggctcgacatcgg caaggtgtgggtcgcggacgacggcgccgcggtggcggtctggaccacgccggagagcgtcgaagcggggg cggtgttcgccgagatcggcccgcgcatggccgagttgagcggttcccggctggccgcgcagcaacagatg gaaggcctcctggcgccgcaccggcccaaggagcccgcgtggttcctggccaccgtcggagtatcgcccga ccaccagggcaagggtctgggcagcgccgtcgtgctccccggagtggaggcggccgagcgcgccggggtgc ccgccttcctggagacctccgcgccccgcaacctccccttctacgagcggctcggcttcaccgtcaccgcc gacgtcgaggtgcccgaaggaccgcgcacctggtgcatgacccgcaagcccggtgcctgaacccagctttc ttgtacaaagtggtgatggccggccgcttcgagcagacatgataagatacattgatgagtttggacaaacc acaactagaatgcagtgaaaaaaatgctttatttgtgaaatttgtgatgctattgctttatttgtaaccat tataagctgcaataaacaagttaacaacaacaattgcattcattttatgtttcaggttcagggggaggtgt gggaggttttttaaagcaagtaaaacctctacaaatgtggtagatatcaagcttaaacaagaatctctagt ttctttcttgcttttacttttacttccttaatactcaagtacaattttaatggagtactttttactttt actcaagtaagattctagccagatacttttacttttaattgagtaaaattttccctaagtacttgtacttt cacttgagtaaaatttttgagtactttttacacctctgggcgctcttccgcttcctcgctcactgactcgc tgcgctcggtcgttcggctgcggcgagcggtatcagctcactcaaaggcggtaatacggttatccacagaa tcaggggataacgcaggaaagaacatgtgagcaaaaggccagcaaaaggccaggaaccgtaaaaaggccgc gttgctggcgtttttccataggctccgcccccctgacgagcatcacaaaaatcgacgctcaagtcagaggt ggcgaaacccgacaggactataaagataccaggcgtttccccctggaagctccctcgtgcgctctcctgtt ccgaccctgccgcttaccggatacctgtccgcctttctcccttcgggaagcgtggcgctttctcatagctc acgctgtaggtatctcagttcggtgtaggtcgttcgctccaagctgggctgtgtgcacgaaccccccgttc agcccgaccgctgcgccttatccggtaactatcgtcttgagtccaacccggtaagacacgacttatcgcca ctggcagcagccactggtaacaggattagcagagcgaggtatgtaggcggtgctacagagttcttgaagtg gtggcctaactacggctacactagaagaacagtatttggtatctgcgctctgctgaagccagttaccttcg gaaaaagagttggtagctcttgatccggcaaacaaaccaccgctggtagcggtggtttttttgtttgcaag cagcagattacgcgcagaaaaaaaggatctcaagaagatcctttgatcttttctacggggtctgacgctca gtggaacgaaaactcacgttaagggattttggtcatgagattatcaaaaaggatcttcacctagatccttt taaattaaaaatgaagttttaaatcaatctaaagtatatatgagtaaacttggtctgacagttaccaatgc ttaatcagtgaggcacctatctcagcgatctgtctatttcgttcatccatagttgcctgactccccgtcgt gtagataactacgatacgggagggcttaccatctggccccagtgctgcaatgataccgcgagatccacgct caccggctccagatttatcagcaataaaccagccagccggaagggccgagcgcagaagtggtcctgcaact ttatccgcctccatccagtctattaattgttgccgggaagctagagtaagtagttcgccagttaatagttt gcgcaacgttgttgccattgctacaggcatcgtggtgtcacgctcgtcgtttggtatggcttcattcagct ccggttcccaacgatcaaggcgagttacatgatcccccatgttgtgcaaaaaagcggttagctccttcggt cctccgatcgttgtcagaagtaagttggccgcagtgttatcactcatggttatggcagcactgcataattc tcttactgtcatgccatccgtaagatgcttttctgtgactggtgagtactcaaccaagtcattctgagaat agtgtatgcggcgaccgagttgctcttgcccggcgtcaatacgggataataccgcgccacatagcagaact ttaaaagtgctcatcattggaaaacgttcttcggggcgaaaactctcaaggatcttaccgctgttgagatc cagttcgatgtaacccactcgtgcacccaactgatcttcagcatcttttactttcaccagcgtttctgggt gagcaaaaacaggaaggcaaaatgccgcaaaaaagggaataagggcgacacggaaatgttgaatactcata ctcttcctttttcaatattattgaagcatttatcagggttattgtctcatgagcggatacatatttgaatg tatttagaaaaataaacaaataggggttccgcgcacatttccccgaaaagtgccacctgacgtctaagaaa ccattattatcatgacattaacctataaaaataggcgtatcacgaggccctttcgtc |
| 3 | Tol2-Toransposase | caactttgtatagaaaagttgctcgacattgattattgactagttattaatagtaatcaattacggggtca |
| | pDNA | ttagttcatagcccatatatggagttccgcgttacataacttacggtaaatggcccgcctggctgaccgcc caacgacccccgcccattgacgtcaataatgacgtatgttcccatagtaacgccaatagggactttccatt gacgtcaatgggtggagtatttacggtaaactgcccacttggcagtacatcaagtgtatcatatgccaagt acgccccctattgacgtcaatgacggtaaatggcccgcctggcattatgcccagtacatgaccttatggga ctttcctacttggcagtacatctacgtattagtcatcgctattaccatggtcgaggtgagccccacgttct gcttcactctccccatctcccccccctccccacccccaattttgtatttatttattttttaattattttgt gcagcgatgggggcgggggggggggggggcgcgcgccaggcggggcggggcggggcgaggggcggggcgg ggcgaggcggagaggtgcggcggcagccaatcagagcggcgcgctccgaaagtttccttttatggcgaggc ggcggcggcggcggccctataaaaagcgaagcgcgcggcgggcgggagtcgctgcgcgctgccttcgcccc gtgccccgctccgccgccgcctcgcgccgcccgccccggctctgactgaccgcgttactcccacaggtgag cgggcgggacggcccttctcctccgggctgtaattagcgcttggtttaatgacggcttgtttcttttctgt ggctgcgtgaaagccttgaggggctccgggagggccctttgtgcggggggagcggctcggggggtgcgtgc gtgtgtgtgtgcgtggggagcgccgcgtgcggctccgcgctgcccggcggctgtgagcgctgcgggcgcgg cgcggggctttgtgcgctccgcagtgtgcgcgaggggagcgcggccgggggcggtgccccgcggtgcgggg ggggctgcgaggggaacaaaggctgcgtgcggggtgtgtgcgtgggggggtgagcagggggtgtgggcgcg tcggtcgggctgcaaccccccctgcacccccctccccgagttgctgagcacggcccggcttcgggtgcggg gctccgtacggggcgtggcgcggggctcgccgtgccgggcggggggtggcggcaggtgggggtgccgggcg gggcggggccgcctcgggccggggagggctcgggggaggggcgcggcggcccccggagcgccggcggctgt cgaggcgcggcgagccgcagccattgccttttatggtaatcgtgcgagagggcgcagggacttcctttgtc ccaaatctgtgcggagccgaaatctgggaggcgccgccgcaccccctctagcgggcgcggggcgaagcggt gcggcgccggcaggaaggaaatgggcggggagggccttcgtgcgtcgccgcgccgccgtccccttctccct ctccagcctcggggctgtccgcggggggacggctgccttcgggggggacggggcagggcggggttcggctt ctggcgtgtgaccggcggctctagagcctctgctaaccatgttcatgccttcttctttttcctacagctcc tgggcaacgtgctggttattgtgctgtctcatcattttggcaaagaattgcaagtttgtacaaaaaagcag gcttaatacgactcactatagggccaccatggaggaagtatgtgattcatcagcagctgcgagcagcaca gtccaaaatcagccacaggatcaagagcacccgtggccgtatcttcgcgaattcttttctttaagtggtgt aaataaagattcattcaagatgaaatgtgtcctctgtctcccgcttaataaagaaatatcggccttcaaaa gttcgccatcaaacctaaggaagcatattgagagaatgcacccaaattacctcaaaaactactctaaattg acagcacagaagagaaagatcgggacctccacccatgcttccagcagtaagcaactgaaagttgactcagt tttcccagtcaaacatgtgtctccagtcactgtgaacaaagctatattaaggtacatcattcaaggacttc atcctttcagcactgttgatctgccatcatttaaagagctgattagtacactgcagcctggcatttctgtc attacaaggcctactttacgctccaagatagctgaagctgctctgatcatgaaacagaaagtgactgctgc catgagtgaagttgaatggattgcaaccacaacggattgttggactgcacgtagaaagtcattcattggtg taactgctcactggatcaaccctggaagtcttgaaagacattccgctgcacttgcctgcaaaagattaatg ggctctcatacttttgaggtactggccagtgccatgaatgatatccactcagagtatgaaatacgtgacaa ggttgtttgcacaaccacagacagtggttccaactttatgaaggctttcagagtttttggtgtggaaaaca atgatatcgagactgaggcaagaaggtgtgaaagtgatgacactgattctgaaggctgtggtgagggaagt gatggtgtggaattccaagatgcctcacgagtcctggaccaagacgatggcttcgaattccagctaccaaa acatcaaaagtgtgcctgtcacttacttaacctagtctcaagcgttgatgcccaaaaagctctctcaaatg agcactacaagaaactctacagatctgtctttggcaaatgccaagctttatggaataaaagcagccgatcg gctctagcagctgaagctgttgaatcagaaagccggcttcagcttttaaggccaaaccaaacgcggtggaa ttcaacttttatggctgttgacagaattcttcaaatttgcaaagaagcaggagaaggcgcacttcggaata tatgcacctctcttgaggttccaatgtttaatccagcagaaatgctgttcttgacagagtggccaacaca atgcgtccagttgcaaaagtactcgacatcttgcaagcggaaacgaatacacagctggggtggctgctgcc tagtgtccatcagttaagcttgaaacttcagcgactccaccattctctcaggtactgtgacccacttgtgg atgccctacaacaaggaatccaaacacgattcaagcatatgtttgaagatcctgagatcatagcagctgcc atccttctccctaaatttcggacctcttggacaaatgatgaaaccatcataaaacgaggcatggactacat cagagtgcatctggagcctttggaccacaagaaggaattggccaacagttcatctgatgatgaagattttt tcgcttctttgaaaccgacaacacatgaagccagcaaagagttggatggatatctggcctgtgtttcagac accagggagtctctgctcacgtttcctgctatttgcagcctctctatcaagactaatacacctcttcccgc atcggctgcctgtgagaggcttttcagcactgcaggattgcttttcagccccaaaagagctaggcttgaca ctaacaattttgagaatcagcttctactgaagttaaatctgaggttttacaactttgagtagacccagctt tcttgtacaaagtggtgatggccggccgcttcgagcagacatgataagatacattgatgagtttggacaaa ccacaactagaatgcagtgaaaaaaatgctttatttgtgaaatttgtgatgctattgctttatttgtaacc attataagctgcaataaacaagttaacaacaacaattgcattcattttatgtttcaggttcagggggaggt gtgggaggttttttaaagcaagtaaaacctctacaaatgtggtagcggccgcggcgctcttccgcttcctc |
| | | gctcactgactcgctgcgctcggtcgttcggctgcggcgagcggtatcagctcactcaaaggcggtaatac ggttatccacagaatcaggggataacgcaggaaagaacatgtgagcaaaaggccagcaaaaggccaggaac cgtaaaaaggccgcgttgctggcgtttttccataggctccgcccccctgacgagcatcacaaaaatcgacg ctcaagtcagaggtggcgaaacccgacaggactataaagataccaggcgtttccccctggaagctccctcg tgcgctctcctgttccgaccctgccgcttaccggatacctgtccgcctttctcccttcgggaagcgtggcg ctttctcatagctcacgctgtaggtatctcagttcggtgtaggtcgttcgctccaagctgggctgtgtgca cgaaccccccgttcagcccgaccgctgcgccttatccggtaactatcgtcttgagtccaacccggtaagac acgacttatcgccactggcagcagccactggtaacaggattagcagagcgaggtatgtaggcggtgctaca gagttcttgaagtggtggcctaactacggctacactagaagaacagtatttggtatctgcgctctgctgaa gccagttaccttcggaaaaagagttggtagctcttgatccggcaaacaaaccaccgctggtagcggtggtt tttttgtttgcaagcagcagattacgcgcagaaaaaaaggatctcaagaagatcctttgatcttttctacg gggtctgacgctcagtggaacgaaaactcacgttaagggattttggtcatgagattatcaaaaaggatctt cacctagatccttttaaattaaaaatgaagttttaaatcaatctaaagtatatatgagtaaacttggtctg acagttaccaatgcttaatcagtgaggcacctatctcagcgatctgtctatttcgttcatccatagttgcc tgactccccgtcgtgtagataactacgatacgggagggcttaccatctggccccagtgctgcaatgatacc gcgagacccacgctcaccggctccagatttatcagcaataaaccagccagccggaagggccgagcgcagaa gtggtcctgcaactttatccgcctccatccagtctattaattgttgccgggaagctagagtaagtagttcg ccagttaatagtttgcgcaacgttgttgccattgctacaggcatcgtggtgtcacgctcgtcgtttggtat ggcttcattcagctccggttcccaacgatcaaggcgagttacatgatcccccatgttgtgcaaaaaagcgg ttagctccttcggtcctccgatcgttgtcagaagtaagttggccgcagtgttatcactcatggttatggca gcactgcataattctcttactgtcatgccatccgtaagatgcttttctgtgactggtgagtactcaaccaa gtcattctgagaatagtgtatgcggcgaccgagttgctcttgcccggcgtcaatacgggataataccgcgc cacatagcagaactttaaaagtgctcatcattggaaaacgttcttcggggcgaaaactctcaaggatctta ccgctgttgagatccagttcgatgtaacccactcgtgcacccaactgatcttcagcatcttttactttcac cagcgtttctgggtgagcaaaaacaggaaggcaaaatgccgcaaaaaagggaataagggcgacacggaaat gttgaatactcatactcttcctttttcaatattattgaagcatttatcagggttattgtctcatgagcgga tacatatttgaatgtatttagaaaaataaacaaataggggttccgcgcacatttccccgaaaagtgccacc tgacgtctaagaaaccattattatcatgacattaacctataaaaataggcgtatcacgaggccctttcgtc ggcgcgccgcggccgc |
| 4 | Tol2 Seq PTEN | ctgtcactctcttagaacg |
| 5 | Tol2 Seq CMV | ttaccgtaagttatgtaacgc |

## Claims

1. A method of producing genomic DNA as a calibration reference material to be used in analyzing a mutation in a target sequence, comprising
a step of introducing a mutated target sequence and an internal standard sequence into genomic DNA.

2. The method according to claim 1, wherein the introduced target sequence and internal standard sequence are derived from a recombinant vector.

3. The method according to claim 2, wherein the recombinant vector is a transposon vector.

4. The method according to claim 2 or 3, wherein the recombinant vector is produced by a method including
1) a step of cleaving a recombinant vector with a first restriction enzyme,
wherein the recombinant vector has a nucleic acid sequence in which a first restriction enzyme recognition sequence, a second restriction enzyme recognition sequence, and a first restriction enzyme recognition sequence are arranged in that order,
the two first restriction enzyme recognition sequences are arranged such that cleavage sites for two first restriction enzymes are located on the sides opposite to the second restriction enzyme recognition sequence, and
the first restriction enzyme recognition sequence is a Type IIS restriction enzyme recognition sequence, and the second restriction enzyme recognition sequence is a restriction enzyme recognition sequence different from the first restriction enzyme recognition sequence; and
2) a step of ligating a double-stranded nucleic acid containing a region composed of a polymorphic nucleotide in a target sequence and its 5'-side and 3'-side flanking sequences together with their complementary sequences.

5. The method according to any one of claims 1 to 3, wherein the introduced sequence further contains a selection marker.

6. The method according to any one of claims 1 to 3, wherein the mutation is a polymorphism.

7. The method according to any one of claims 1 to 3, wherein the analysis is a companion diagnostic.

8. The method according to any one of claims 1 to 3, wherein the calibration reference material is used to validate a companion diagnostic agent.

9. Genomic DNA produced by the method according to any one of claims 1 to 3.

10. A cell comprising one or more copies of genomic DNA into which a mutated target sequence and an internal standard sequence have been introduced.

11. The cell according to claim 10, wherein the chromosome number is diploid.

12. A method of analyzing a mutation in a target sequence, comprising:
1) a step of subjecting genomic DNA into which a mutated target sequence and an internal standard sequence have been introduced to an amplification reaction; and
2) a step of calculating the copy numbers of the introduced internal standard sequence and a different internal standard sequence inherent to the genomic DNA.

13. The method according to claim 12, further comprising a step of calculating a variant allele frequency (VAF) based on the copy number.

14. The method according to claim 13,
wherein the VAF is a percentage value calculated from the following formula: (copy number of introduced internal standard sequence-copy number of different internal standard sequence inherent to genomic DNA)/copy number of introduced internal standard sequence.

15. The method according to any one of claims 12 to 14, which is used to evaluate the sensitivity of a reagent for detecting a mutation.

16. The method according to claim 15, wherein the evaluation of the sensitivity is used as an index for validating the reagent for detecting the mutation.

17. The method according to any one of claims 12 to 14, wherein the amplification reaction is performed by a PCR.

18. The method according to claim 17, wherein the PCR is a droplet digital PCR.
